# EUROPEAN PATENT APPLICATION

(11) **EP 4 353 191 A1**
(43) Date of publication of application: **17.04.2024**
(21) Application number: 22819203.5
(22) Date of filing: 15.04.2022
(51) Int. Cl.: A61F 2/24

(54) **VALVE CLAMPING SYSTEM**

(30) Priority: 08.06.2021 CN 202110637346; 08.09.2021 CN 202111048210; 08.09.2021 CN 202111048222
(71) Applicant: Shanghai Shenqi Medical Technology Co., Ltd., Shanghai 201201 (CN)
(72) Inventor: WANG, Sen, Shanghai 201201 (CN); SONG, Rui, Shanghai 201201 (CN); WANG, Zhichen, Shanghai 201201 (CN); DAI, Zhihao, Shanghai 201201 (CN); HUANG, Zhongrong, Shanghai 201201 (CN)
(74) Representative: Cabinet Laurent & Charras
(86) International application number: PCT/CN2022/087004
(87) International publication number: WO 2022/257600

(57) **Abstract**

A valve clamping system, which comprises an adjustment mechanism and a locking mechanism; the adjustment mechanism comprises a proximal clamping piece (1), a distal clamping piece (2), a central base body (3), and a bottom base body; the proximal clamping piece (1) is engaged on the central base body (3), and the distal clamping piece (2) is rotatably connected on the central base body (3) and the bottom base body so as to move close to or move away from the proximal clamping piece (1); the central base body (3) has an accommodating cavity, and opposing through holes are provided on a side wall of the central base body (3); the locking mechanism comprises a fixing piece A (6), a fixing piece B (7), a push rod (8), and a control member (9); the fixing piece A (6) and the fixing piece B (7) are caught in mutual abutment within the accommodating cavity, and the push rod (8) is located within the accommodating cavity and passes through the fixing piece A (6) and the fixing piece B (8) and connects to the bottom base body; and the control member (9) and the fixing piece B (7) are jointly configured to adjust the position of the fixing piece A (6) within the accommodating cavity. Stable clamping of a valve leaflet is consequently achieved, the present valve clamping system is easy to operate, and surgical risk can be lowered.

## Description

### TECHNICAL FIELD

The present application relates to a field of medical devices, and in particular, to a valve clamping system.

### BACKGROUND

A tricuspid valve is located in an opening between a right atrium and a right ventricle. The tricuspid valve opens to allow blood from the right atrium to fill the right ventricle. When the right ventricle contracts to pump blood into lungs, the tricuspid valve closes to prevent blood from flowing back into the right atrium. When the tricuspid valve is not completely closed, some blood leaks back into the right atrium through the tricuspid valve with each contraction of the right ventricle. This is referred to as tricuspid regurgitation.

In recent years, transcatheter interventional therapy for tricuspid regurgitation has become a research focus and a challenge in the field of heart valve disease treatment. The current valvular intervention technique is based on an edge-to-edge surgical principle, and delivers a valve clamp to a target tricuspid valve, and enables the valve leaflets of the tricuspid valve to be clamped through the relative opening and closing of clamp plates, so as to relatively fix the valve leaflets of the tricuspid valve, and thus reduce a gap between the valve leaflets and reduce tricuspid regurgitation. Compared with a mitral valve, the tricuspid valve has a larger annulus and valve area, varies most, and has a more fragile annulus tissue, and more thin leaflets and chordae tendineae; the adjacent structures of the tricuspid valve are complex, the surrounding tissues are prone to injury. The annulus is in semilunar saddle shape, irregular in shape and most prone to dilation, and the degree of tricuspid regurgitation is most likely to be affected by the volume load. In addition, the right ventricle has abundant myocardial trabeculae, fleshy columns, and chordae tendineae, and its free wall is weak; and the right ventricle is prone to thrombosis due to low blood flow rate, which poses a great challenge to the design and application of surgical devices. Therefore, there is an urgent need for a valve clamping system that can effectively control the opening and closing of the clamp plates and reduce the difficulty and risk of surgery.

### SUMMARY

Accordingly, the present application discloses a valve clamping system that can effectively control the opening and closing of clamp plates and reduce the difficulty and risk of surgery.

The present application provides a valve clamping system including: an adjusting mechanism including a proximal clamp plate, a distal clamp plate, a central seat body, and a base body, the proximal clamp plate being connected to the central seat body, the distal clamp plate being connected to the central seat body and the base body, respectively, the central seat body including a receiving cavity, and a via hole extending through a sidewall of the central seat body; and a locking mechanism including a fixing piece A, a fixing piece B, a push rod, and a control member, the fixing piece A and the fixing piece B abutting against each other and being arranged in the receiving cavity, the push rod being located in the receiving cavity, extending through the fixing piece A and the fixing piece B and being connected to the base body, the control member being capable of extending through the via hole, and the control member bypassing the fixing piece A and the fixing piece B on either one of sides of the push rod.

The control member and the fixing piece B are together configured to adjust a position of the fixing piece A within the receiving cavity. The push rod is fixed relative to the fixing piece A when the fixing piece A is in a first position. The push rod is movable relative to the fixing piece A when the fixing piece A is not in the first position.

Further, a stop protrusion is disposed on an inner wall of the receiving cavity. The fixing piece A and the fixing piece B are both elastic pieces. The fixing piece B is configured to cause the fixing piece A to abut against the stop protrusion and be not in the first position. The control member bypasses the fixing piece A and the fixing piece B on a side of the push rod adjacent to the stop protrusion. The control member is pulled to enable the fixing piece A to be in the first position.

Further, the control member is a metal wire or a polymer wire. The control member extends in an axial direction of the push rod. The control member is capable of bypassing the fixing piece A and the fixing piece B on the side of the push rod adjacent to the stop protrusion. The fixing piece A is connected to the control member only at an end of the fixing piece A adjacent to the stop protrusion.

Further, a stop protrusion is disposed on an inner wall of the receiving cavity. The fixing piece A is an elastic piece. The fixing piece B is configured to cause the fixing piece A to abut against the stop protrusion and be in the first position. The control member bypasses the fixing piece A and the fixing piece B on a side of the push rod away from the stop protrusion. The control member is pulled to enable the fixing piece A to be not in the first position.

Further, the fixing piece A includes a support foot A engaged to an outer sidewall of the central seat body; and the fixing piece B includes a support foot B engaged to the outer sidewall of the central seat body.

Further, a surface roughness of the push rod and a surface roughness at an abutment portion of the fixing piece A abutting against the push rod are both greater than or equal to 1.6 µm.

Further, a blocking membrane is disposed on the proximal clamp plate and/or the distal clamp plate.

Further, a mounting portion is disposed on the central seat body. An end of the distal clamp plate is rotatably connected to the central seat body through the mounting portion.

Further, the base body includes a connecting rod and a base. A portion of the distal clamp plate not connected to the central seat body is rotatably connected to the connecting rod. The push rod is connected to the base.

Further, the adjusting mechanism further includes a pulling wire connected to the proximal clamp plate. The pulling wire is configured to bring a portion of the proximal clamp plate not abutting against the central seat body closer to or farther away from the central seat body.

Further, the adjusting mechanism further includes a push-pull wire connected to the push rod. The push-pull wire is configured to enable a movement of the push rod within the receiving cavity.

Further, the valve clamping system further includes a delivering mechanism configured to deliver the adjusting mechanism and the locking mechanism to a target position. The delivering mechanism includes a fixing member at an end proximate to an operator. The fixing member is configured to be detachably connected to the control member.

Further, the proximal clamp plate is an elastic clamp plate. The proximal clamp plate is in snap-fit with and disposed on the central seat body. The distal clamp plate is rotatably connected to the central seat body and the base body to be closer to or farther away from the proximal clamp plate.

In the valve clamping system of the present application, the proximal clamp plate has elasticity and is connected to the central seat body. The distal clamp plate is rotatably connected to the central seat body and the base body to be closer to or farther away from the proximal clamp plate. The clamping of a tricuspid valve can be realized through the cooperation of the distal clamp plate and the proximal clamp plate. The central seat body includes a receiving cavity. The fixing piece A and the fixing piece B abut against each other and are engaged into the receiving cavity. The push rod is located in the receiving cavity, extends through the fixing piece A and the fixing piece B, and is connected to the base body. The control member bypasses the fixing piece A and the fixing piece B on either one of the sides of the push rod. The control member and the fixing piece B can adjust a position of the fixing piece A in the receiving cavity. A relative fixation of the push rod and the fixing member A, i.e., locking of the position of the distal clamp plate, can be realized by adjusting the position of fixed piece A, which can ensure stable clamping of the tricuspid valve by the distal clamp plate and the proximal clamp plate. The valve clamping system is simple and easy to operate and reduces the difficulty of clamping, which reduces the difficulty of surgery for the operator and the risk of surgery for the patient.

### BRIEF DESCRIPTION OF THE DRAWINGS

In order to more clearly illustrate the technical solutions in the embodiments of the present application or the prior art, a brief description is given below for the drawings referred in the description of the embodiments or the prior art. Obviously, the drawings in the following description are merely some embodiments of the application. For those of ordinary skill in the art, other drawings can also be obtained based on the disclosed drawings without involving any inventive effort.
FIG. 1 is a perspective schematic view of a valve clamping system according to Embodiment I of the present application.
FIG. 2 is a schematic view of a fixing piece assembled with a central seat body according to Embodiment I of the present application.
FIG. 3 is a schematic view of a locking mechanism in a locked state according to Embodiment I of the present application.
FIG. 4 is a schematic view of a locking mechanism in an unlocked state according to Embodiment I of the present application.
FIG. 5 is a schematic view of a locking mechanism in a locked state according to Embodiment II of the present application.
FIG. 6 is a schematic view of a locking mechanism in an unlocked state according to Embodiment II of the present application.

Illustration For Numeral References:
1, proximal clamp plate; 2, distal clamp plate; 3, central seat body; 31, stop protrusion; 32, mounting portion; 4, base; 5, connecting rod; 6, fixing piece A; 7, fixing piece B; 8, push rod; and 9, control member.

### DETAILED DESCRIPTION

In order to make the technical problems to be solved, the adopted technical solutions, and the achieved technical effects in the present application clearer, the technical solutions of the present application will be further illustrated in combination with the drawings and specific implementations. It should be understood that specific embodiments described herein are only intended to illustrate the present application but not intended to limit the present application. It should be noted that, for the convenience of description, the drawings only show some parts related to the present application rather than the entire structure.

In the description of the present application, unless otherwise expressly specified and defined, the terms "connected", "coupled" and "fixed" should be understood in a broad sense, for example, fixedly connected or detachably connected, or integrated; or mechanically connected or electrically connected; or directly connected or indirectly connected through an intermediate medium, or in an interior communication or mutual interaction relationship between two elements. For those of ordinary skill in the art, the specific meanings of the above-described terms in the present application may be understood according to specific circumstances.

In the present application, unless otherwise expressly specified and defined, the first feature, when being referred to as being "on" or "under" the second feature, may be in direct contact with the second feature, or may be in indirect contact with the second feature through other features therebetween. Moreover, the first feature, when being referred to as being disposed "on", "above" and "over" the second feature, may be disposed right above or obliquely above the second feature, or simply disposed at a level higher than the second feature. The first feature, when being referred to as being disposed "under", "below" and "beneath" the second feature, may be disposed directly below or obliquely below the second feature, or simply disposed at a level lower than the second feature.

In the description of the embodiments, the terms "up", "down", "left", "right", etc., indicate the orientations or position relationships based on the orientations or position relationships shown in the drawings, and are used only for the convenience of describing and simplifying operations, rather than indicating or implying that a device or unit referred to must have a specific orientation, must be constructed and operated in a specific orientation, therefore they should not be construed as limitations on the present application. In addition, the terms "first" and "second" are only used to distinguish between descriptions and have no special meaning.

The technical solutions of the present application will be further illustrated in combination with the drawings and specific implementations.

### Embodiment I

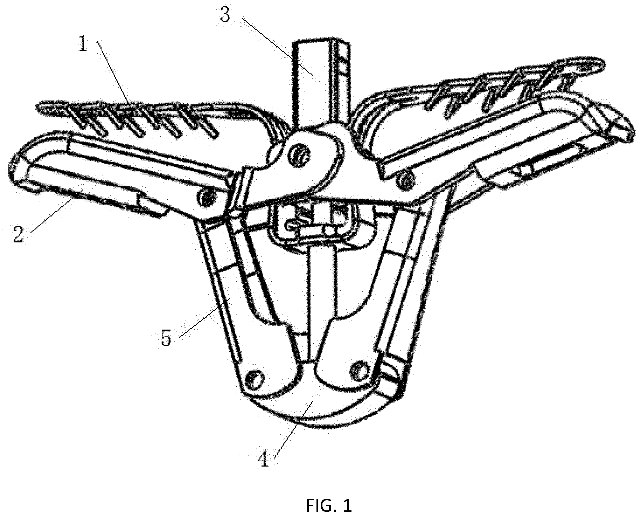

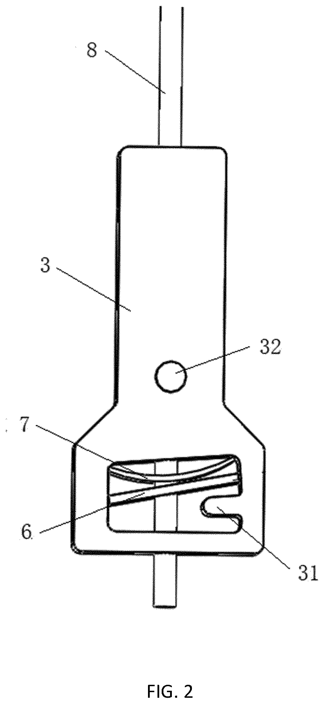

As shown in FIGS. 1 to 4, the present embodiment provides a valve clamping system including an adjusting mechanism and a locking mechanism. The adjusting mechanism includes a proximal clamp plate 1, a distal clamp plate 2, a central seat body 3, and a base body. The proximal clamp plate 1 is in snap-fit with the central seat body 3. The distal clamp plate 2 is connected to the central seat body 3 and the base body. The central seat body 3 includes a receiving cavity. A via hole extends through a sidewall of the central seat body 3. The locking mechanism includes a fixing piece A 6, a fixing piece B 7, a push rod 8, and a control member 9. The fixing piece A 6 and the fixing piece B 7 abut against each other and are arranged in the receiving cavity. The push rod 8 is located in the receiving cavity, and extends through the fixing piece A 6 and the fixing piece B 7, and is then connected to the base body. The control member 9 can extend through the via hole, and bypass the fixing piece A 6 and the fixing piece B 7 on either one of the sides of the push rod 8. The control member 9 and the fixing piece B 7 can adjust a position of the fixing piece A 6 in the receiving cavity. The push rod 8 is fixed relative to the fixing piece A 6 when the fixing piece A 6 is in a first position; and the push rod 8 is movable relative to the fixing piece A 6 when the fixing piece A 6 is not in the first position.

The tricuspid valve can be clamped through the cooperation of the distal clamp plate 2 and the proximal clamp plate 1. Specifically, before the valve clamping system is delivered to the designated position, the distal clamp plate 2 and the proximal clamp plate 1 are both in a closed state, while after being delivered to the designated site, in order to clamp the tricuspid valve, it is required for the distal clamp plate 2 and the proximal clamp plate 1 to be in an opened state, so that one valve leaflet can be fixed between the distal clamp plate 2 and the proximal clamp plate 1 on one side, and another valve leaflet can be fixed between the distal clamp plate 2 and the proximal clamp plate 1 on another side, thereby facilitating subsequent suturing of relative leaflets.

It should be noted that the connection between the proximal clamp plate 1 and the central seat body 3, and the connections between the distal clamp plate 2 and the central seat body 3 and between the distal clamp plate 2 and the base body may be fixed, rotatable or removable connections such as a snap-fit connection, a threaded connection, a riveted connection, or a bonded connection, and which are not limited herein, and can be flexibly adapted and configured according to the actual needs.

In a specific embodiment, the proximal clamp plate 1 is an elastic clamp plate. In addition, the distal clamp plate 1 is rotatably connected to the central seat body 3 and the base body to be closer to or away from the proximal clamp plate 1.

Specifically, in order to ensure the fixation effect of the valve clamping system for the tricuspid valve, that is when the distal clamp plate 2 and the proximal clamp plate 1 are utilized to clamp the tricuspid valve, it should be ensured that the relative positions of the distal clamp plate 2 and the proximal clamp plate 1 are fixed. Optionally, the proximal clamp plate 1 is of a Q shape, and a fitting portion may be formed in the proximal clamp plate 1, so that by means of an interference fit between the proximal clamp plate 1 and the central seat body 3, the fitting portion of the proximal clamp plate 1 is fixed relative to the central seat body 3. Two branches of the proximal clamp plate 1 that are away from the central seat body 3 have elasticity, so that the branches may be in the opened state when not subjected to an external force, and may be in a closed state when subjected to an external force. Optionally, the closing and opening of the distal clamp plate 2 is controlled by the push rod 8. A through hole extend through the central seat body 3 in a direction parallel to the sidewall of the central seat body 3. The through hole is in communication with the receiving cavity. The push rod 8 is inserted into the through hole, and the control of the distal clamp plate 2 is realized by the movement of the push rod 8 in the through hole.

Specifically, a mounting portion 32 is disposed on the central seat body 3 of the valve clamping system according to present embodiment. An end of the distal clamp plate 2 is rotatably connected to the central seat body 3 through the mounting portion 32. Optionally, the mounting portion 32 is configured as circular protrusions provided on opposing outer sidewalls of the central seat body 3. The distal clamp plate 2 is provided with mounting holes, so that the circular protrusions can be inserted into the mounting holes. Optionally, the mounting portion 32 is a pin or a fixing rod extending through the central seat body 3, which is secured to the central seat body 3 by means of welding or screwing, and which, similar to the aforementioned circular protrusions, can be inserted into the mounting holes on the distal clamp plate 2.

In addition to the end of the distal clamp plate 2 being rotatably connected to the central seat body 3, the remaining parts of the distal clamp plate 2 also need to be rotatably connected to the base body. In present embodiment, the base body includes a connecting rod 5 and a base 4. A portion of the distal clamp plate 2 that is not connected to the central seat body 3 is rotatably connected to the connecting rod 5. The push rod 8 is connected to the base 4. In this way, it can be realized that, by pushing the push rod 8, the distal clamp plate 2 gradually opens when the base 4 moves away from the central seat body 3, and the distal clamp plate 2 gradually closes when the base 4 moves approaching the central seat body 3. Thus, if the distal clamp plate 2 needs to be fixed in a certain position, the push rod 8 is required to be fixed in a relative position in the through hole.

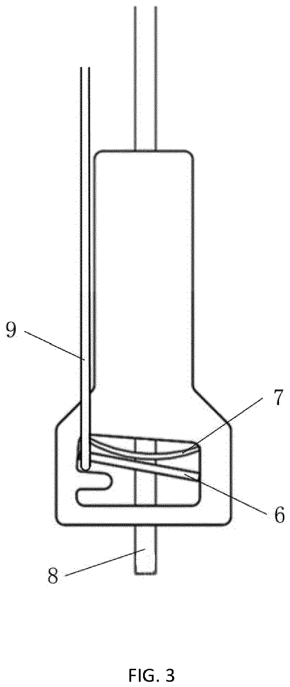

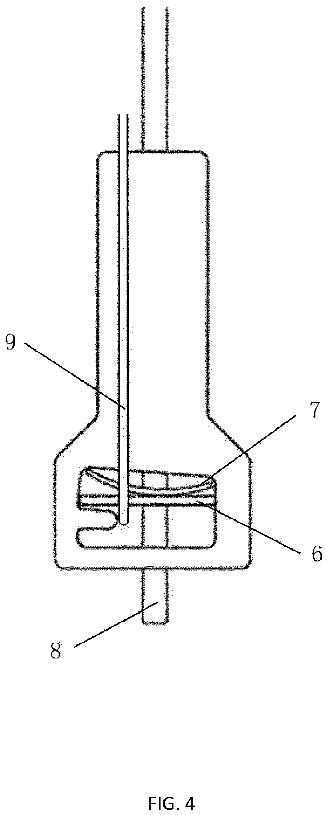

In order to fix the position of the distal clamp plate 2 as described above, in present embodiment, a stop protrusion 31 is disposed on the inner wall of the receiving cavity. The fixing piece A 6 and the fixing piece B 7 are both configured as elastic pieces. The fixing piece B 7 is used to cause the fixing piece A 6 to abut against the stop protrusion 31 and be not in the first position. The control member 9 bypasses the fixing piece A 6 and the fixing piece B 7 on a side of the push rod 8 adjacent to the stop protrusion 31. The fixing piece A 6 can be in the first position by pulling the control member 9. Specifically, as shown in FIGS. 3 and 4, the control member 9 is disposed on a left side of the push rod 8 and bypasses the fixing piece A 6 and the fixing piece B 7. The fixing piece B 7 is disposed over the fixing piece A 6. The forces between the fixing piece B 7 and the fixing piece A 6 are balanced, so that the fixing piece B 7 and the fixing piece A 6 can abut against each other and be placed in the receiving cavity. Optionally, the control member 9 is a metal wire made of stainless steel, aluminum alloy or the like, or a polymer wire having a certain rigidity. Optionally, the metal wire or polymer wire extends in an axial direction of the push rod, and the fixing piece A is connected to the control member only at an end of the fixing piece A adjacent to the stop protrusion.

In present embodiment, the fixing piece A 6 can be horizontally placed in the receiving cavity by the force of gravity and the elastic force exerted by the fixing piece B 7. In this case, the push rod 8 extends through the fixing piece A 6 and the fixing piece B 7, and can be moved relative to the fixing piece A 6. In another embodiment, it is not limited to that the fixing piece A 6 can only be horizontally placed in the receiving cavity, it is not limited to that the fixing piece A 6 can only be horizontally placed in the receiving cavity, as long as it is ensured that the fixing piece A 6 is not in the first position when it is not affected by the force of the control member 9. However, as shown in FIG. 3, if the control member 9 is tensioned so that the fixing piece A 6 is in the first position, the push rod 8 will then be stuck by the fixing piece A 6. Specifically, the central seat body 3, the fixing piece A 6, and the fixing piece B 7 are all provided with circular holes. When the fixing piece A 6 is in the first position, and a projection area of the circular hole of the fixing piece A 6 on a horizontal plane is smaller than a cross-sectional area of the push rod 8, the push rod 8 will be stuck in the circular hole of the fixing piece A 6, that is, the relative positions of the push rod 8 and the fixing piece A 6 are fixed. However, as shown in FIG. 4, if the control member 9 is released, when the fixing piece A 6 is not in the first position, the push rod 8 can be moved through the circular holes of the three aforementioned components to realize the movement of the push rod 8 relative to the fixing piece A 6, so that the position of the distal clamp plate 2 can be adjusted.

Further, in order to ensure the relative position of the fixing piece A 6 and the fixing piece B 7 in the receiving cavity, in addition to make ensure that both the fixing piece A 6 and the fixing piece B 7 are elastic pieces. Optionally, the fixing piece A 6 includes a support foot A. The support foot A is engaged to an outer sidewall of the central seat body 3. A plurality of support feet A is provided. The friction between the fixing piece A 6 and the central seat body 3 is increased through the interference fit between the plurality of support feet A and the outer sidewall of the central seat body, so that when the position of the fixing piece A 6 is changed by an external force, the position change can be smoother and steadier. Optionally, the fixing piece B 7 includes a support foot B. The support foot B is engaged to the outer sidewall of the central seat body 3. A plurality of support feet B is provided, and has the similar functions as that of the support feet A.

It also should be noted that the fixing piece B 7 is an elastic piece for the purpose of abutting against the fixing piece A 6, so that the fixing piece A 6 can be in a predetermined position. Therefore, in present embodiment, the fixing piece B 7 and the central seat body 3 are not fixedly connected to each other. However, in another embodiment, the fixing piece B 7 can be fixed to the inner wall of the receiving cavity by welding, alternatively, the fixing piece B 7 and the central seat body 3 can be integrally formed. Optionally, shapes of the bodies of the fixing piece A 6 and the fixing piece B 7 include, but are not limited to, rectangle, square, or circle. In present embodiment, the shapes of the fixing pieces are mainly considered to be configured to adapt to the shape of the receiving cavity. That is, it is required to ensure that the fixing pieces can abut against the inner sidewall of the receiving cavity. Optionally, the fixing piece A 6 and the fixing piece B 7 may be made of a metal material including, but not limited to, a nickel-titanium alloy, a stainless steel, or an aluminum alloy.

Furthermore, while ensuring the smoothness of the pushing of the push rod 8, it is also required to ensure the firmness of the relative fixation of the fixing piece A 6 and the push rod 8, so as to cause the distal clamp plate 2 and the proximal clamp plate 1 to clamp the valve leaflet more stable. In present embodiment, a surface roughness of the push rod 8 and a surface roughness at an abutment portion of the fixing piece A 6 abutting against the push rod 8 are both greater than or equal to 1.6 µm.

Optionally, blocking membranes are disposed on the proximal clamp plate 1 and the distal clamp plate 2 respectively. The blocking membrane may partially or completely cover the surface of the proximal clamp plate 1 or the distal clamp plate 2. The blocking membrane may form a friction portion on the surface of the clamp plate to increase the adhesion force. Specifically, the blocking membrane may enable rapid endothelialization of the clamp plate. The rapid endothelialization may, on the one hand, effectively reduce an anticoagulant administration period, shorten the treatment time, reduce the treatment cost, and alleviate the pain of the patient, and, on the other hand, reduce the risk of forming device thrombosis, and improve the safety of the device-based implantation. Optionally, the blocking membrane is fixed to the proximal clamp plate 1 and the distal clamp plate 2 by welding. Specifically, one or more points on the blocking membrane may be heat welded to the proximal clamp plate 1 and the distal clamp plate 2. Optionally, the blocking membrane is bound and secured to the proximal clamp plate 1 and distal clamp plate 2 by cotton or polymer filaments.

Specifically, the blocking membrane can be made of a polymer membrane by means of heating and cutting through a heat-setting mold, or formed by weaving. The blocking membrane formed by weaving is generally made of cotton or polymer filaments, with a filament diameter in a range from 0.01 mm to 0.02 mm. 2 to 32 strands of filaments are used to weave, and the weaving density (Picks Per Inch, PPI) is in a range from 30-100. The weave is realized by pressing n strands onto m strands, where n and m may or may not have the same value. In addition, a plurality of apertures is formed in the blocking membrane, and shapes of the apertures include, but are not limited to, circle, rectangle, etc. Specifically, a maximum radius of an aperture size is in a range from 15 µm to 600 µm, and an aperture percentage is in a range from 10% to 90%.

Optionally, the blocking membrane may be made of a material including, but not limited to, one of or any combination of polyethylene terephthalate (PET), polyester, polyurethane, expanded polytetrafluoroethylene (ePTFE), cotton products, silicon, or various biocompatible polymers or fibers. Optionally, the blocking membrane may also incorporate drugs, antibiotics, antithrombotic agents, or antiplatelet agents, such as heparin or warfarin sodium, which may be impregnated into the blocking membrane or coated on the blocking membrane, and which may cover the valve leaflet or be absorbed by a bloodstream when the clamp plates clamp the valve leaflet.

Optionally, the proximal clamp plate 1 and the distal clamp plate 2 may be assembled from separate components made of biocompatible materials. The separate components may be made of the same or different materials including, but not limited to, stainless steel, nitinol, titanium, tantalum, metal alloys, or polymers, or alternatively may be made of biocompatible materials that can be absorbed by surrounding tissues or dissolved into the bloodstream after the implantation.

In summary, when using the valve clamping system according to present embodiment, before the valve clamping system reaches a lesion site, the proximal clamp plate 1 and the distal clamp plate 2 are in the closed state, the control member 9 is in a tensioned state, and the fixing piece A 6 is in the first position. When the valve clamping system is delivered to a designated position, the control member 9 can be in a released state so that the fixing piece A 6 is not in the first position. The push rod 8 can then be moved towards a side away from the base 4 in the through hole, and at this time, the distal clamp plate 2 can be opened relative to the central seat body 3 under the pulling of the connecting rod 5. When the distal clamp plate 2 is opened at the lesion position located below the valve, it is also required to release the control of the two branches of the proximal clamp plate 1. After being released, the two branches of the proximal clamp plate 1 can be opened towards the direction approaching the distal clamp plate 2. At this time, the distal clamp plate 2 and the proximal clamp plate 1 gradually approach each other to clamp the valve leaflet located therebetween. In addition, when the above-described clamping action is completed, the control member 9 may be tensioned, so that the fixing piece A 6 is in the first position, and the relative fixation of the push rod 8 and the fixing piece A 6 is achieved, thereby locking the distal clamp plate 2. Further, if there is a case where the tricuspid valve is not clamped firmly or the valve clamping system needs to be taken out, the control member 9 can be released again so that the fixing piece A 6 is not in the first position. In this case, the push rod 8 can be moved again, and the position of the distal clamp plate 2 can be readjusted, ready for subsequent operations.

In coordination with the above, the adjusting mechanism further includes a pulling wire connected to the proximal clamp plate 1. The pulling wire is configured to bring a portion of the proximal clamp plate 1 not abutting against the central seat body 3 closer to or farther away from the central seat body 3. It should be understood that, in the present embodiment, the pulling wire is used to control the two branches of the proximal clamp plate 1, so as to enable the proximal clamp plate 1 to be switched back and forth between the closed state and the opened state. Specifically, wire cavities are formed in the two branches of the proximal clamp plate 1 respectively. An end of the pulling wire is disposed in the wire cavity. A deflection of the two branches of the proximal clamp plate 1 with respect to the central seat body 3 can be realized by pulling another end of the pulling wire.

Optionally, the adjusting mechanism further includes a push-pull wire connected to the push rod 8. The push-pull wire is configured to enable a movement of the push rod 8 within the receiving cavity. Specifically, the push-pull wire is fixedly connected to an end of the push rod 8 away from the base 4. When the fixing piece A 6 is not in the first position, the movement of the push rod 8 within the through hole can be enabled by maneuvering the push-pull wire. The fixing means of the push-pull wire and the push rod 8 can be a threaded connection, riveted connection, etc.

Optionally, the valve clamping system further includes a delivering mechanism configured for delivering the adjusting mechanism and the locking mechanism to a target position. The delivering mechanism includes a fixing member at an end proximate to an operator. The fixing member is configured to be detachably connected to the control member. The detachable connection may be a threaded connection, a snap-fit connection, a hinged connection, etc.

### Embodiment II

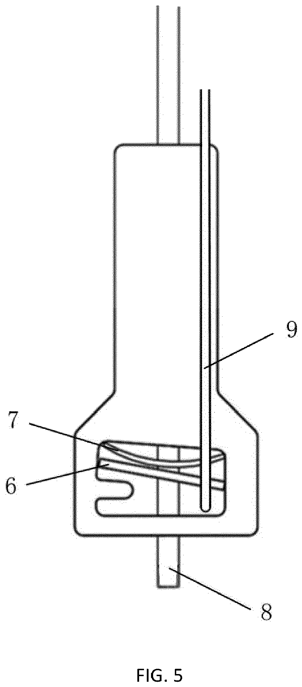

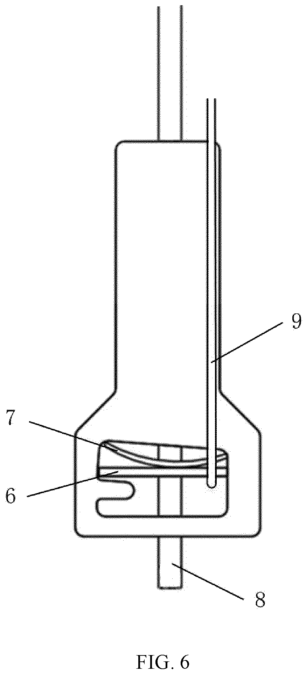

The present embodiment discloses a valve clamping system. The valve clamping system in the present embodiment differs from the valve clamping system in embodiment I in that, as shown in FIGS. 5 and 6, at least the fixing piece B 7 is an elastic piece. The fixing piece B 7 is configured to cause the fixing piece A 6 to abut against the stop protrusion 31, and to be in the first position. The control member 9 bypasses the fixing piece A 6 and the fixing piece B 7 on a side of the push rod 8 away from the stop protrusion 31, and the control member 9 can be pulled to prevent the fixing piece A 6 from being in the first position. Optionally, the fixing piece B 7 and the fixing piece A 6 are both elastic pieces. However, the fixing piece A 6, when subjected to gravity and the elastic force exerted by the fixing piece B 7, is different from that in Embodiment I. As shown in FIG. 5, when releasing the control member 9, the fixing piece A 6 cannot be placed horizontally within the receiving cavity, but is pressed against in the first position by the fixing piece B 7. At this time, the push rod 8 would be stuck by the fixing piece A 6, i.e., the position of the distal clamp plate 2 would be fixed. However, as shown in FIG. 6, when the control member 9 is pulled, the fixing piece A 6 may be moved away from the first position by an external force, at this time the push rod 8 may slide relative to the fixing piece A 6. That is, pushing or pulling the push rod 8 may adjust the distal clamp plate 2 to be in the opened state or in the closed state. Optionally, only the fixing piece B 7 is an elastic piece. The fixing piece A 6 is subjected to gravity and the elastic force exerted by the fixing piece B 7 to be in the first position, and at this time, the push rod 8 is fixed relative to the fixing piece A 6. Certainly, the fixing piece A 6 may also move away from the first position under the action of the control member 9, and at this time, the push rod 8 is movable relative to the fixing piece A 6.

In summary, when using the valve clamping system according to present embodiment, before the valve clamping system reaches a lesion site, the proximal clamp plate 1 and the distal clamp plate 2 are in a closed state, the control member 9 is in a released state, and the fixing piece A 6 is in the first position. When the valve clamping system is delivered to a designated position, the control member 9 can be in a tensioned state so that the fixing piece A 6 is not in the first position. The push rod 8 can then be moved towards a side away from the base 4 in the through hole, and at this time the distal clamp plate 2 can be opened relative to the central seat body 3 under the pulling of the connecting rod 5. When the distal clamp plate 2 is opened at the lesion position located below the valve, it is also required to release the control of the two branches of the proximal clamp plate 1. After being released, the two branches of the proximal clamp plate 1 can be opened towards the direction approaching the distal clamp plate 2. At this time, the distal clamp plate 2 and the proximal clamp plate 1 gradually approach each other to clamp the tricuspid valve therebetween. When the above-described clamping action is completed, the control member 9 may be released, so that the fixing piece A 6 is in the first position, and the relative fixation of the push rod 8 and the fixing piece A 6 can be achieved, thereby locking the distal clamping piece 2. Further, if there is a case where the tricuspid valve is not clamped firmly or the valve clamping system needs to be taken out, the control member 9 can be tensioned again so that the fixing piece A 6 is not in the first position. In this case, the push rod 8 can be moved again, and the position of the distal clamp plate 2 can be readjusted, ready for subsequent operations.

In addition to the above, the rest components of the valve clamping system according to present embodiment have the same configuration as those of the valve clamping system in Embodiment I, and which will not be repeated herein.

The valve clamping system of the present application can be used for clamping not only the tricuspid valve but also the mitral valve. In the valve clamping system of the present application, the proximal clamp plate has elasticity and is in snap-fit with the central seat body. The distal clamp plate is rotatably connected to the central seat body and the base body to be closer to or farther away from the proximal clamp plate. The clamping of the mitral valve can be realized through the cooperation of the distal clamp plate and the proximal clamp plate. The central seat body includes the receiving cavity. The fixing piece A and the fixing piece B abut against each other and are engaged into the receiving cavity. The push rod is located in the receiving cavity, extends through the fixing piece A and the fixing piece B, and is connected to the base body. The control member bypasses the fixing piece A and the fixing piece B on either one of the sides of the push rod. The control member and the fixing piece B can adjust a position of the fixing piece A in the receiving cavity. The relative fixation of the push rod and the fixing piece A, i.e., the locking of the position of the distal clamp plate, can be realized by adjusting the position of fixed piece A, which can ensure stable clamping of the mitral valve by the distal clamp plate and the proximal clamp plate. The valve clamping system is simple and easy to operate and reduces the difficulty of clamping, which reduces the difficulty of surgery for the operator and the risk of surgery for the patient.

The above implementations only illustrate the basic principles and characteristics of the present application. The present application is not limited by the above implementations. Various modifications and variants can be made for the present application, and these modifications and variants, without departing from the spirit and scope of the present application, all fall within the scope of protection required by the present application. The scope of protection claimed by the present application shall be defined by the attached claims and their equivalents.

## Claims

1. A valve clamping system, **characterized by**, comprising:
an adjusting mechanism comprising a proximal clamp plate, a distal clamp plate, a central seat body, and a base body, the proximal clamp plate being connected to the central seat body, the distal clamp plate being connected to the central seat body and the base body, respectively, the central seat body comprising a receiving cavity, and a via hole extending through a sidewall of the central seat body; and
a locking mechanism comprising a fixing piece A, a fixing piece B, a push rod, and a control member, the fixing piece A and the fixing piece B abutting against each other and being arranged in the receiving cavity, the push rod being located in the receiving cavity, extending through the fixing piece A and the fixing piece B and being connected to the base body, the control member being capable of extending through the via hole, and bypassing the fixing piece A and the fixing piece B on either one of sides of the push rod,
wherein the control member and the fixing piece B are together configured to adjust a position of the fixing piece A within the receiving cavity; the push rod is fixed relative to the fixing piece A when the fixing piece A is in a first position; and the push rod is movable relative to the fixing piece A when the fixing piece A is not in the first position.

2. The valve clamping system according to claim 1, wherein a stop protrusion is disposed on an inner wall of the receiving cavity; the fixing piece A and the fixing piece B are both elastic pieces, the fixing piece B is configured to cause the fixing piece A to abut against the stop protrusion and be not in the first position; the control member bypasses the fixing piece A and the fixing piece B on a side of the push rod adjacent to the stop protrusion; and the control member is pulled to enable the fixing piece A to be in the first position.

3. The valve clamping system according to claim 2, wherein the control member is a metal wire or a polymer wire; the control member extends in an axial direction of the push rod; the control member is capable of bypassing the fixing piece A and the fixing piece B, and the fixing piece A on the side of the push rod adjacent to the stop protrusion; and the fixing piece A is connected to the control member only at an end of the fixing piece A adjacent to the stop protrusion.

4. The valve clamping system according to claim 1, wherein a stop protrusion is disposed on an inner wall of the receiving cavity; the fixing piece A is an elastic piece; the fixing piece B is configured to cause the fixing piece A to abut against the stop protrusion and be in the first position; the control member bypasses the fixing piece A and the fixing piece B on a side of the push rod away from the stop protrusion; and the control member is pulled to enable the fixing piece A to be not in the first position.

5. The valve clamping system according to claim 1, wherein the fixing piece A comprises a support foot A engaged to an outer sidewall of the central seat body; and the fixing piece B comprises a support foot B engaged to the outer sidewall of the central seat body.

6. The valve clamping system according to claim 1, wherein a surface roughness of the push rod and a surface roughness at an abutment portion of the fixing piece A abutting against the push rod are both greater than or equal to 1.6 µm.

7. The valve clamping system according to claim 1, wherein a blocking membrane is disposed on the proximal clamp plate and/or the distal clamp plate.

8. The valve clamping system according to claim 1, wherein a mounting portion is disposed on the central seat body, and an end of the distal clamp plate is rotatably connected to the central seat body through the mounting portion.

9. The valve clamping system according to claim 1, wherein the base body comprises a connecting rod and a base; a portion of the distal clamp plate not connected to the central seat body is rotatably connected to the connecting rod; and the push rod is connected to the base.

10. The valve clamping system according to claim 1, wherein the adjusting mechanism further comprises a pulling wire connected to the proximal clamp plate; and the pulling wire is configured to bring a portion of the proximal clamp plate not abutting against the central seat body closer to or farther away from the central seat body.

11. The valve clamping system according to claim 1, wherein the adjusting mechanism further comprises a push-pull wire connected to the push rod; and the push-pull wire is configured to enable a movement of the push rod within the receiving cavity.

12. The valve clamping system according to claim 1, further comprising a delivering mechanism configured to deliver the adjusting mechanism and the locking mechanism to a target position; the delivering mechanism comprises a fixing member at an end proximate to an operator; and the fixing member is configured to be detachably connected to the control member.

13. The valve clamping system according to claim 1, wherein the proximal clamp plate is an elastic clamp plate; the proximal clamp plate is in snap-fit with and disposed on the central seat body; and the distal clamp plate is rotatably connected to the central seat body and the base body to be closer to or farther away from the proximal clamp plate.
